(19) Europäisches Patentamt
European Patent Office
Office européen des brevets

(11) Veröffentlichungsnummer : **0 326 074 B1**

# (12) EUROPÄISCHE PATENTSCHRIFT

(45) Veröffentlichungstag der Patentschrift :
**01.04.92 Patentblatt 92/14**

(51) Int. Cl.$^5$ : **C07J 41/00,** G01N 33/74

(21) Anmeldenummer : **89101186.8**

(22) Anmeldetag : **24.01.89**

## (54) Neue Haptenderivate und davon abgeleitete Hapten-Protein-Konjugate.

(30) Priorität : **25.01.88 DE 3802060**
**29.09.88 DE 3833149**

(43) Veröffentlichungstag der Anmeldung :
**02.08.89 Patentblatt 89/31**

(45) Bekanntmachung des Hinweises auf die Patenterteilung :
**01.04.92 Patentblatt 92/14**

(84) Benannte Vertragsstaaten :
**AT BE CH DE ES FR GB GR IT LI LU NL SE**

(56) Entgegenhaltungen :
**EP-A- 0 114 011**
**DE-A- 2 354 253**
**DE-A- 2 814 776**

(73) Patentinhaber : **BOEHRINGER MANNHEIM GMBH**
**Patentabteilung, Abt. E Sandhofer Strasse 112-132 Postfach 31 01 20**
**W-6800 Mannheim 31 Waldhof (DE)**

(72) Erfinder : **Huber, Erasmus, Dr.rer.nat.**
**Bürgerplatz 18**
**W-8046 Garching (DE)**
Erfinder : **Klein, Christian, Dr-rer.nat.**
**Blütenstrasse 16**
**W-8120 Weilheim (DE)**
Erfinder : **Pappert, Gunter, Dr.**
**Beringerweg 10**
**W-8132 Tutzing (DE)**
Erfinder : **Hallermayer, Klaus, Dr.rer.nat.**
**Barthstrasse 31**
**W-8000 München 2 (DE)**

(74) Vertreter : **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**W-8000 München 86 (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Haptenderivate, und davon abgeleitete Hapten-Protein-Konjugate.

In der klinischen Diagnostik werden in zunehmendem Maße Bestimmungsverfahren nach dem Prinzip des Immunoassays durchgeführt, die sich durch besondere Empfindlichkeit auszeichnen. Für diese Immunoassays gibt es sehr viele Verfahrensvarianten. Für viele Zwecke werden z.B. kompetitive Assays durchgeführt. Dabei konkurrieren die zu bestimmende Substanz und eine zu der Probe zugegebene bekannte Menge an zu bestimmender Substanz, die mit einem Enzym markiert ist, um einen Antikörper.

Es besteht daher ein Bedarf an Konjugaten aus einem Markierungsenzym und einer mit der zu bestimmenden Substanz bzw. einem Antikörper spezifisch bindefähigen Verbindung, wobei das Konjugat nur mit der zu bestimmenden Substanz bzw. dem Antikörper reagieren darf und keine Kreuzreaktivitäten mit anderen in der Probelösung vorhandenen Verbindungen aufweisen soll, um das Ergebnis nicht zu verfälschen. Da die spezifisch bindende Substanz und das Markierungsenzym häufig nicht direkt aneinander gebunden werden, sondern über eine Verknüpfungsgruppierung (Spacer) gekuppelt werden, kann die Affinität der Antikörper zu dem Spacer zu Problemen führen.

Weiterhin besteht ein großer Bedarf an spezifischen Antikörpern. Im allgemeinen werden Antikörper hergestellt, indem man ein Immunogen wie ein Antigen oder Hapten in geeigneter Form mehrmals in einem zu Antikörperbildung befähigten Organismus injiziert. Haptene sind definitionsgemäß Moleküle mit einem Molekulargewicht von weniger als 1000 Dalton, die allein nicht immunogen wirksam sind, jedoch durch Bindung an ein Protein immunogen werden. Zur Immunisierung wird das Hapten mit einem immunogenen Molekül, wie z.B. einem Serumprotein, konjugiert. Injiziert man ein derartiges Konjugat, so bildet der Organismus neben Anti-Protein-Antikörpern die gewünschten Anti-Hapten-Antikörper. Die gebildeten Antikörper werden dann aus dem Organismus gewonnen. Auf diese Weise können polyklonale Antikörper gewonnen werden.

Auch zur Herstellung monoklonaler Antikörper muß zuerst eine Immunisierung eines geeigneten Organismus, in der Regel Mäuse, durchgeführt werden. Durch die üblicherweise mehrmalige Injektion eines Antigens oder Hapten-Konjugats wird eine B-Zelle dazu veranlaßt, Antikörper gegen dieses Antigen oder Hapten zu synthetisieren und zu sezernieren. Durch Screening wird dann eine B-Zelle, die die gewünschten Antikörper produziert, aus der Milz isoliert und durch Fusionierung mit einer Myelomzelle immortalisiert. Diese Zelle erzeugt dann ständig dieselben monoklonalen Antikörper.

Zur Bindung von Haptenen, die Keto- oder Aldehydfunktionen besitzen, an ein Protein ist es eine übliche Methode, die Carbonylfunktion mit O-(Carboxymethyl)-hydroxylamin zum Oxim umzusetzen und über die freie Carboxylfunktion an ε-Aminogruppen des hochmolekularen Trägermoleküls zu kuppeln (vgl. P.K. Grover und W.D. Odell, J.Steroid. Biochem. 8 (1976) 121). Die Kupplungsprodukte können dann zur Erzeugung von Antikörpern gegen die Haptenkomponente oder als Tracer in Immunoassays verwendet werden (vgl. K. Leubke und B. Nieuweboer, "Immunologische Teste für niedermolekulare Wirkstoffe", Thieme Verlag, Stuttgart 1978).

Bei solchen üblicherweise zur Immunisierung verwendeten Konjugaten, bei denen das Hapten über eine Verknüpfungsgruppierung (Brücke) an das immunogene Protein gebunden ist, treten nun häufig Kreuzreaktionen mit dem Brückenmolekülteil auf und die erhaltenen Antikörper zeigen eine zum Teil recht hohe Affinität für die im Immunogen vorhandenen Brückenstrukturen. Dies führt in immunologischen Tests oft zu Problemen, weil ein Teil der Bindungsaktivität der Antikörper gegen den Linker, d.h. gegen das Brückenmolekül zwischen Hapten und Trägerprotein im Immunogen, gerichtet ist. Um diesen Effekt, der oft zu nachteiligen Auswirkungen bei der Entwicklung von Immunoassays, insbesondere für niedrigkonzentrierte Haptene, führt, zu umgehen, bedient man sich bei der Herstellung von Hapten-Enzym-Konjugaten vorzugsweise der Methode der heterologen Linker (vgl. z.B. B.K. Van Weemen und A.H.W.M. Schuurs, Immunochem. 12 (1975) 667; H. Hosada et al., Chem. Pharm. Bull. 34 (1986) 2105), d.h. die Verknüpfungsgruppierung im Konjugat wird gegenüber dem Immunogen verfremdet, d.h. strukturell abgewandelt.

Wird nun zur Erzeugung von Antikörpern ein Immunogen verwendet, in dem ein Hapten mit einer Keto- oder Aldehydfunktion über O-(Carboxymethyl)-hydroxylamin an den Lysin-Rest eines Trägerproteins gekuppelt ist, so waren für einen heterologen Linker bei gleicher Verknüpfungsstelle des Haptens im Immunogen und Enzym-Konjugat bisher folgende Möglichkeit bekannt:

a) die Verwendung von Glycin-Hydrazid als Brücke (vgl. R. Mueller et al., Hoppe-Seylers Z. Physiol. Chem. 357 (1976) 1007). Dabei reagiert die Hydrazid-Funktion zum Hydrazon und über die freie Aminogruppe des Linkers erfolgt anschließend die Kupplung des Haptenderivats an eine Carboxylfunktion des Enzyms.

b) Die Umwandlung der Carbonyl-Funktion in eine Hydroxyl-Funktion und Kupplung über eine Ether- oder Ester-Funktion an das Brückenmolekül (vgl. K. Luebke und B. Nieuweboer, l. c. und B.K. Van Weemen und A.H.W.M. Schuurs, l. c.).

Beide Methoden besitzen jedoch Nachteile: die Methode a) ist auf Enzyme beschränkt, die freie Carboxylgruppen besitzen, die außerdem nicht am reaktiven Zentrum des Enzyms sitzen dürfen. Die Kupplungsreaktion erfolgt unter Verwendung von Carbodiimid, wobei es im allgemeinen zu unerwünschten Vernetzungsreaktionen des Enzyms kommen kann. Bei der Methode b) wird das Hapten chemisch so weitgehend verändert, daß die Bindung zum Antikörper nachteilig beeinflußt wird.

Aufgabe der vorliegenden Erfindung war es daher, Hapten-Protein-Konjugate zur Verfügung zu stellen, die zur Bildung spezifisch gegen das Hapten gerichteter Antikörper führen und bei der Verwendung in Immunoassays zu einer spezifischen Bindung von Antikörpern am Hapten führen, ohne daß Kreuzreaktivitäten mit dem Brückenmolekülteil auftreten. Diese Aufgabe wird mit der vorliegenden Erfindung gelöst.

Gegenstand der Erfindung sind Haptenderivate der allgemeinen Formel (I)

$$\begin{array}{c} \quad\quad\quad\quad\quad\;\; R^1 \\ \quad\quad\quad\quad\quad\;\; | \\ Hap{=}N{-}O{-}C{-}COOH \\ \quad\quad\quad\quad\quad\;\; | \\ \quad\quad\quad\quad\quad\;\; R^2 \end{array} \qquad (I)$$

worin Hap der aus einem Keto- oder Aldehydgruppen tragenden Hapten durch Abspaltung einer Oxogruppe gebildete Rest ist, und $R^1$ und $R^2$ gleich oder verschieden sind und eine Niederalkylgruppe mit 1 bis 7, insbesondere 1 bis 3 Kohlenstoffatomen bedeuten, und ein Rest $R^1$ oder $R^2$ auch ein Wasserstoffatom sein kann

Eine Niederalkylgruppe $R^1$ und $R^2$ mit 1 bis 7, vorzugsweise 1 bis 3 Kohlenstoffatomen kann geradkettig oder verzweigt sein; vorzugsweise ist die Niederalkylgruppe geradkettig, und ist z.B. Methyl, Ethyl, n-Propyl, n-Butyl, n-Pentyl, n-Hexyl, oder n-Heptyl.

Der Haptenrest in der allgemeinen Formel (I) leitet sich vorzugsweise von einem Steroidhormon ab, wie z.B. von Testosteron, Progesteron, Oestradiol (über 6-Keto-Oestradiol) oder von Oestriol (über 6-Keto-Oestriol).

Die erfindungsgemäßen Haptenderivate der allgemeinen Formel (I) können hergestellt werden, indem man in einem eine Keto- oder Aldehydgruppe tragenden Hapten eine Keto- oder Aldehydgruppe mit einer Verbindung der Formel (II)

$$\begin{array}{c} \quad\quad\quad\quad\; R^1 \\ \quad\quad\quad\quad\; | \\ H_2N{-}O{-}C{-}COOH \\ \quad\quad\quad\quad\; | \\ \quad\quad\quad\quad\; R^2 \end{array} \qquad . (II)$$

worin $R^1$ und $R^2$ die für Formel (I) angegebene Bedeutung besitzen, in das entsprechende Oximderivat überführt.

Die Überführung der Keto- oder Aldehydgruppen in das Oxim durch Umsetzung des Haptens mit einer Verbindung der Formel (II) kann auf für eine solche Oximbildung allgemein bekannte Weise erfolgen. Die Kondensation zum Oxim erfolgt z.B. durch Umsetzung mit dem entsprechenden Hydrochlorid oder Hydrobromid des Carboxyalkoxyamins der Formel (II) unter alkalischen Bedingungen.

Weist das Hapten keine geeignete funktionelle Gruppe (Oxogruppe) auf, so kann eine solche zur Kondensation eingeführt werden (z.B. in 6-Stellung eines Steroidhormons); die Kondensation des Haptens mit der Verbindung der Formel (II) muß in jedem Fall so erfolgen, daß sein Epitop nach der Kondensation frei zugänglich bleibt. Nach der Umsetzung mit dem Hapten werden gegebenenfalls in das Hapten eingeführte Sch...

Die Hapten-Protein-Konjugate der Formel (III) können hergestellt werden, indem man ein erfindungsgemäßes Haptenderivat der Formel (I) mit einem Protein E-$NH_2$, worin -$NH_2$ eine ε-Aminogruppe eines Lysinrestes bedeutet, umsetzt.

Die Umsetzung des Haptenderivats der Formel (I) mit der epsilon-Aminogruppe kann selektiv auf an sich bekannte Weise durchgeführt werden durch Überführung der Carboxylgruppe des Haptenderivates der Formel (I) in einen aktivierten Ester, insbesondere in den entsprechenden N-Hydroxysuccinimidester (vgl. K. Leubke und B. Nieuweboer, l.c.).

In den Hapten-Protein-Konjugaten der Formel (III) bedeutet der Haptenrest Hap vorzugsweise einen der für die Haptenderivate der Formel (I) bevorzugt genannten Reste.

Die bevorzugte Bedeutung des Proteinrestes E richtet sich insbesondere nach der vorgesehenen Verwendung des erfindungsgemäßen Hapten-Protein-Konjugats. Zur Immunisierung von zur Antikörperbildung geeigneten Organismen ist das Protein ein Immunogenprotein, insbesondere eines der üblicherweise verwendeten immunogenen Carrier-Proteine, wie z.B. Edestin oder Rinderserumalbumin. Für den Einsatz in Immunoassays werden vorzugsweise die üblicherweise in Immunoassays verwendeten Enzyme verwendet, und insbesondere die Markerenzyme wie z.B. β-Galactosidase oder Peroxidase.

In einer Ausführungsform der Erfindung werden Hapten-Protein-Konjugate zur Verfügung gestellt, in denen das Hapten ein Steroidhormon ist. Der Nachweis von Steroidhormonen wie Oestrogenen, Testosteron, Cortisonen und Herzglucosiden, denen allen das Steroid-Grundgerüst gemeinsam ist, ist für die Diagnostik sehr wichtig. Die Zurverfügungstellung von Antikörpern gegen Steroidhormone sowie von Steroidhormon-Enzym-Konjugaten für die Durchführung von Immunoassays ist daher wünschenswert. Es hat sich nun als sehr vorteilhaft erwiesen, Steroidhormone gegebenenfalls so zu derivatisieren, daß sie an dem C6-Atom eine Oxogruppe tragen. Diese Oxogruppe kann kann mit den Carboxyalkoxyaminen der Formel (II) zum Haptenderivat (I) umgesetzt werden. Diese Bindung beeinträchtigt nicht das Epitop des Steroidhormons und führt zu keinen unerwünschten Veränderungen des Moleküls. Je nach Verwendungszweck kann dann des Haptenderivat entweder an ein immunogenes Protein oder ein Enzym gekuppelt werden.

Bei der Verwendung für die Immunisierung wird bevorzugt ein Konjugat verwendet, bei dem das Protein ein immunogenes Carrier-Protein, wie z.B. Edestin oder Rinderserumalbumin, ist. Es sind jedoch auch Konjugate, die Enzyme als Protein enthalten, für die Immunisierung geeignet. Das erfindungsgemäße Konjugat wird in den zur Antikörperbildung geeigneten Organismus in Abständen mehrmals injiziert. Das Konjugat bewirkt dann die Bildung von Antikörpern, die in sehr hohem Prozentsatz nur gegen das Hapten gerichtet sind und keine Kreuzreaktivität mit der Verknüpfungsgruppierung aufweisen. Die Antikörper können dann in an sich bekannter Weise aus dem Organismus gewonnen werden. Die erfindungsgemäß verwendeten Hapten-Carrier-Konjugate eignen sich sowohl zur Immunisierung für die Herstellung polyklonaler Antikörper als auch zur Herstellung monoklonaler Antikörper.

In einer weiteren Ausführungsform der vorliegenden Erfindung wird ein erfindungsgemäßes Hapten-Protein-Konjugat der Formel (III) aus einem Hapten, das über die erfindungsgemäße Verknüpfungsgruppierung an ein Enzym gebunden ist, verwendet zur Durchführung von Immunoassays. Das erfindungsgemäße Hapten-Enzym-Konjugat kann dann als Marker (Markierungsenzym) in Bestimmungsverfahren nach dem Prinzip des Immunoassays eingesetzt werden. Das erfindungsgemäße Hapten-Enzym-Konjugat kann z.B. in bekannter Menge einer Probelösung zugesetzt werden und konkurriert dann um einen gegen das Hapten gerichteten Antikörper.

Überraschenderweise gelingt es durch Verwendung eines erfindungsgemäßen Hapten-Protein-Konjugats, Antikörper zu erhalten, die sehr wenig Kreuzreaktivität mit der Brückenverbindung aufweisen. Die erhaltenen Antikörper haben eine hohe Affinität zu dem Hapten. Bei Verwendung der erfindungsgemäßen Hapten-Protein-Konjugate zur Immunisierung werden hohe Antikörpertiter erhalten. Weiterhin sind die erfindungsgemäßen Hapten-Protein-Konjugate besonders gut geeignet zum Einsatz in Immunoassays. Da die Antikörper zu dem Brückenmolekül keine oder nur eine sehr geringe Affinität aufweisen und daher das Hapten sehr spezifisch binden, erhält man mit diesen Konjugaten bei der Verwendung in Immunoassays sehr genaue Ergebnisse.

Die erfindungsgemäßen Haptenderivate der Formel (I) besitzen den Vorteil, daß sie einerseits die Struktur des freien Haptens weitgehend unverändert beinhalten, andererseits im Vergleich zu Derivaten, die z.B. analog mit O-(Carboxymethyl)-hydroxylamin hergestellt wurden, im Brückenmolekül eine charakteristische Änderung aufweisen. Die Verwendung der erfindungsgemäßen Haptenderivate ermöglicht deshalb die Durchführung heterologer Linker-Techniken auf einfache und effektive Weise: für die beiden Anwendungszwecke als

1. Immunogen und
2. Konjugat in der immunologischen Analyse

werden vorzugsweise verschiedene Brücken im erfindungsgemäßen Hapten-Protein-Konjugat verwendet (mindestens ein Rest $R^1$ oder $R^2$ ist verschieden, wobei für einen der beiden Anwendungszwecke auch Derivate, die sich von O-(Carboxymethyl)-hydroxylamin ableiten, d.h. $R^1$ und $R^2$=H, verwendet werden können),

die aber auf der anderen Seite wieder möglichst ähnlich sein sollten, um die Spezifität beizubehalten.

Gegenstand der Erfindung ist deshalb auch die Verwendung eines erfindungsgemäßen Hapten-Protein-Konjugats der Formel (III) zur Immunisierung von zur Antikörperbildung geeigneten Organismen, und als Markierungsenzym in einem Immunoassay.

Die nachfolgenden Beispiele erläutern die Erfindung in Verbindung mit der Abbildung näher.

Fig. 1 zeigt die Ergebnisse der Testosteronbestimmung mit dem erfindungsgemäßen Testosteron-3-dmc-POD-Konjugat im Vergleich zu Testosteron-3-cmo-POD.

Die Verbindungen der Formel (II) sind bekannt, oder lassen sich analog einem Verfahren zur Herstellung der Bekannten Verbindungen herstellen (vgl. K.S. Suresh und R.K. Malkani, Ind. J. Chem. 10 (1972) 1068).

**Beispiele**

Beispiel 1

Herstellung von Testosteron-3-dimethylcarboxymethoxim-POD-Konjugat (Testosteron-3-dmc-POD, Formel (III), $R^1$=$R^2$=Methyl, Hap=Testosteronrest, E-NH=Peroxidaserest).

Die Herstellung erfolgte nach dem folgenden Reaktionsschema:

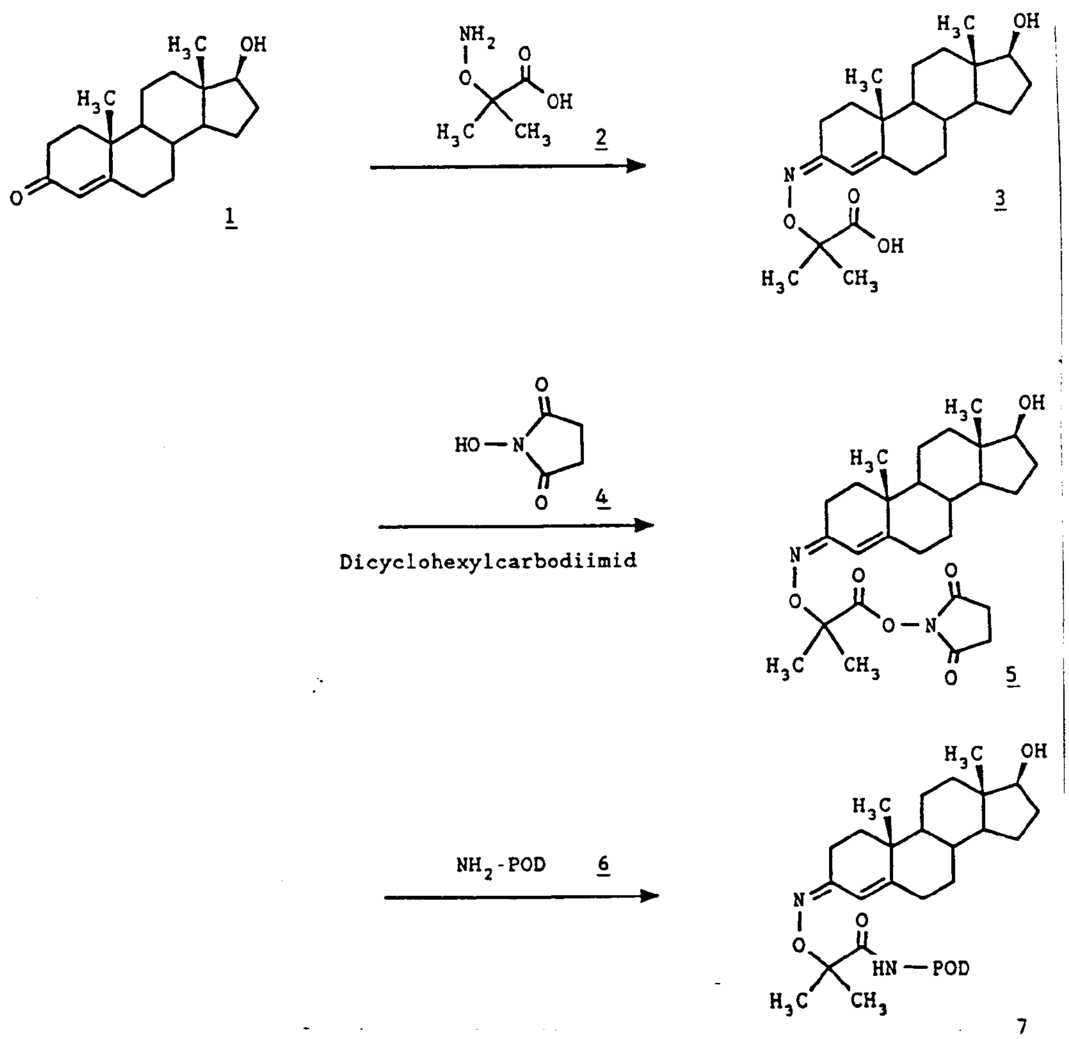


1. Testosteron-3-dimethylcarboxymethoxim (3)

2,88 g (10 mmol) Testosteron (1) werden in 50 ml Methanol gelöst, mit 1,65 ml (20 mmol) Pyrrolidin versetzt und bei 20°C gerührt. In der gelben Lösung tritt nach kurzer Zeit eine Fällung auf. Nach 10 Minuten gibt man 2,1 g (10,5 mmol) 2-Amino-oxi-iso-buttersäure-Hydrobromid (2) (hergestellt nach der Vorschrift von K.S.

Suresh und R.K. Malkani, Ind. J. Chem. 10 (1972) 1068) zu und erwärmt 5 Minuten auf 65°C. Danach wird die Lösung am Wasserstrahlvakuum eingedampft, der Rückstand mit 50 ml 1 mol/l NaOH versetzt und anschließend in 2 l Wasser gelöst. Die fast klare Lösung wäscht man zweimal mit je 250 ml Essigester. Die alkalische Phase wird mit konzentrierter HCl auf pH 1 eingestellt und zweimal mit je 200 ml Essigester extrahiert. Den Essigesterextrakt wäscht man dann mit 0,5 l Wasser, trocknet mit 20 g $Na_2SO_4$ und dampft in Vakuum ein. Man digeriert mit Petrolether, beläßt die Suspension etwa 2 Stunden unter Rühren und saugt das feste Produkt (3) ab. Es wird im Exsikkator über Phosphorpentoxid getrocknet.
Ausbeute: 1,8 g (46% d.Th.).
DC: Kieselgel; Chloroform/Methanol/Eisessig=66/33/1; $R_f$=0,69.

2. Testosteron-3-dimethylcarboxymethoxim-N-hydroxysuccinimidester (5)

0,97 g (2,5 mmol) der Carbonsäure (3) werden mit 0,35 g (3 mmol) N-Hydroxysuccinimid (4) in 20 ml Tetrahydrofuran (THF) gelöst und mit 0,62 g (3 mmol) Dicyclohexylcarbodiimid in 10 ml THF versetzt. Man läßt 3 Stunden bei 20°C rühren, dann wird vom ausgefallenen Dicyclohexylharnstoff abfiltriert und die Lösung im Wasserstrahlvakuum eingedampft. Den Rückstand löst man in 50 ml Dioxan und kühlt die Lösung 16 Stunden bei 4°C. Dann wird filtriert und das Lösungsmittel im Vakuum entfernt. Den öligen Rückstand digeriert man so lange mit 50 ml Diisopropylether, bis er sich vollkommen verfestigt hat. Das Produkt (5) wird abgesaugt und im Exsikkator über Phosphorpentoxid getrocknet.
Ausbeute: 0,86 g (70% d.Th.).
DC: Kieselgel; Chloroform/Methanol/Eisessig=80/19/1; $R_f$=0,70.

3. Testosteron-3-dimethylcarboxymethoxim-POD-Konjugat (7)

35 mg Peroxidase (POD) (6) (EC 1.11.1.7) werden in 3,5 ml 0,1 M Kaliumphosphat (pH 8,0) gelöst, auf 4°C abgekühlt und langsam mit 3,3 ml DMSO (Dimethylsulfoxid) versetzt.

5 mg des vorstehend unter 2. beschriebenen Testosteron-3-dimethylcarboxymethoxim-N-hydroxysuccinimidesters (5) werden in 200 µl DMSO bei Raumtemperatur gelöst und in 6 gleichen Portionen im Abstand von ca. 30 Minuten der kalten POD-Lösung zugesetzt. Der Ansatz wurde über Nacht bei 4°C gerührt.

Für die Abtrennung des freien Esters wird eine Säule (2 cm Durchmesser, 16 cm Höhe) mit Sephadex G 25, fine, (Pharmacia) gepackt und bei 4°C mit 40 mmol/l Kaliumphosphat (pH 6,5) äquilibriert. Die gesamte Konjugatlösung wird bei 4°C mit 1 Säulenvolumen/Stunde auf die Säule aufgezogen und bei gleichem Fluß mit Äquilibrierpuffer eluiert. Das Eluat wird fraktioniert aufgefangen und die rötlich-braun gefärbten Fraktionen gesammelt.

Die vereinigten Konjugat-Fraktionen werden mit Thymol gesättigt und bei 4°C gelagert.

Beispiel 2

Testosteron-Bestimmung mit Testosteron-3-carboxymethoxim (cmo)-POD

Reagenzien:

Testpuffer:

400 µg/ml monoklonaler Antikörper gegen Testosteron (ECACC 85121701)
40 mmol/l Natriumphosphat, pH 6,8

Beladungslösung:

10µg/ml polyklonaler Schaf-Antikörper (IgG) gegen Maus Fc-gamma
20 mmol/l Natriumcarbonatpuffer, pH 9,6

Waschlösung:

250 mg/100 ml Natriumchlorid
1 mg/100 ml Kupfersulfat

Substratlösung:

1,9 mmol/l ABTS® (2,2″-Azino-di-[3-ethyl-benzthiazolinsulfonsäure (6)])-Diammoniumsalz
100 mmol/l Phosphat-Citratpuffer, pH 4,4
3,2 mmol/l Natrium-Perborat

Probe:

Als Probe wurde Testosteron in verschiedenen Konzentrationen in Pufferlösung verwendet:
40 mmol/l Natriumphosphat, pH 7,4
0,9 % Natriumchlorid
0,3 % RSA (Rinderserumalbumin)
0,2 % Pluronic PF68

Zur Durchführung der Bestimmung wurden 1 ml Beschichtungslösung 30 Minuten bei Raumtemperatur in einem Luran-Röhrchen inkubiert. Anschließend wurde zweimal mit Waschlösung gewaschen. Es wurde 1 ml Testpuffer zugegeben, 60 Minuten bei Raumtemperatur inkubiert und zweimal mit Waschlösung gewaschen. Es wurden 50 µl Probe und 1 ml Testosteron 3-cmo-POD (200 mU/ml) in 40 mmol/l Natriumphosphatpuffer, pH 6,8, mit 0,2 % Pluronic PF68 zugegeben, 30 Minuten bei Raumtemperatur inkubiert und zweimal mit Waschlösung gewaschen. Danach wurde 1 ml Substratlösung zugegeben, 30 Minuten bei Raumtemperatur inkubiert und die Extinktion bei 405 nm gemessen.

**Beispiel 3**

Testosteron-Bestimmung mit Testosteron 3-dmc-POD-Konjugat

Die Bestimmung wurde wie in Beispiel 2 beschrieben durchgeführt, wobei anstelle von Testosteron 3-cmo-POD-Konjugat Testosteron 3-dmc-Konjugat eingesetzt wurde.

Die Ergebnisse von Beispiel 2 und 3 sind aus Fig. 1 zu ersehen. Daraus zeigt sich, daß mit dem erfindungsgemäßen Konjugat eine im unteren Konzentrationsbereich wesentlich steilere Eichkurve (Kurve 2) erhalten wird als mit einem cmo-Konjugat (Kurve 1), wodurch für einen Testosteron-Test, unter Verwendung des erfindungsgemäßen Konjugats, eine wesentlich höhere Empfindlichkeit erhalten wird.

**Beispiel 4**

Herstellung von Oestradiol-6-dimethylcarboxymethoxim-POD-Konjugat

Die Herstellung erfolgte nach dem folgenden Reaktionsschema:

Reaktionsschema:

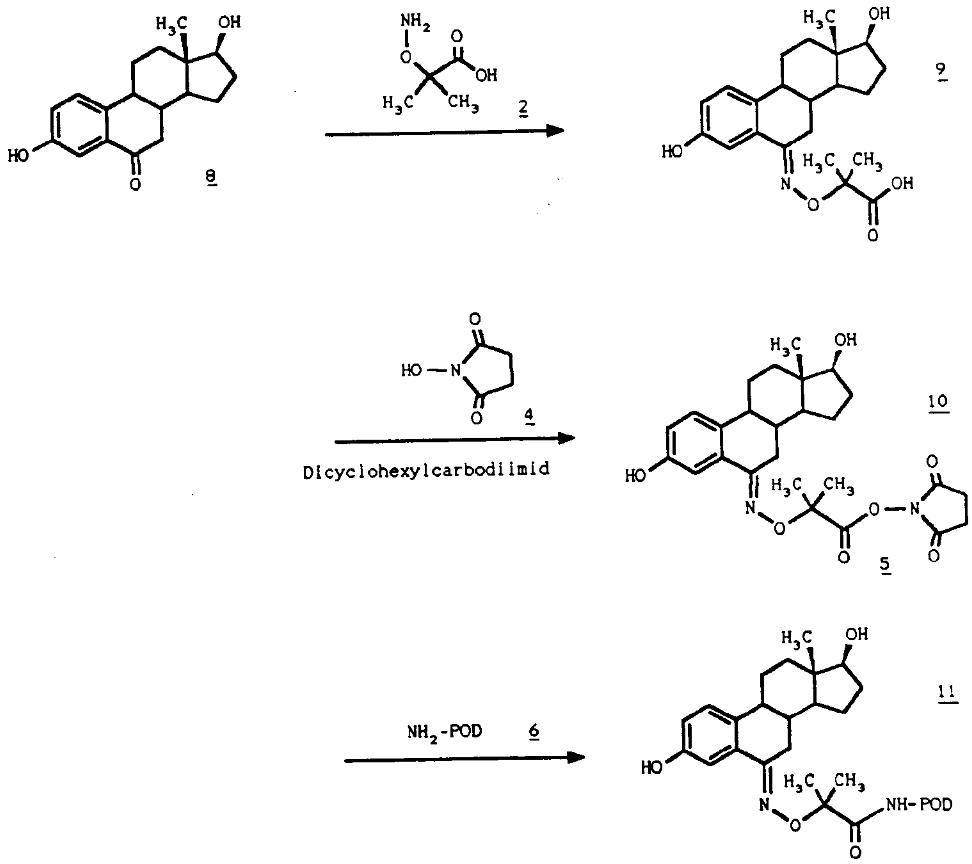


8

9

10

5

11

a) Oestradiol-6-dimethylcarboxymethoxim 9

2,86 g (10 mmol) 6-Oxoestriol 8 wurden in 130 ml Ethanol gelöst und mit 1,7 g (20 mmol) $NaHCO_3$ und 2,1 g (10,5 mmol) 2-Aminooxi-iso-buttersäure-Hydrobromid 2 versetzt. Man erwärmte 30 Minuten auf 65°C, danach wurde die Lösung am Wasserstrahlvakuum eingedampft, der Rückstand mit 50 ml 1 N NaOH versetzt und anschließend in 1 l Wasser gelöst. Die fast klare Lösung wurde zweimal mit je 150 ml Essigester gewaschen. Die alkalische Phase wurde isoliert, mit konzentrierter HCl auf pH 1 eingestellt und mit zweimal je 150 ml Essigester extrahiert. Der Essigesterextrakt wurde mit 0,25 l Wasser gewaschen, getrocknet mit 10 g $Na_2SO_4$ und im Vakuum eingedampft. Man digerierte mit Petrolether, ließ die Suspension etwa 2 Stunden rühren und saugte das feste Produkt 9 ab. Es wurde im Exsikkator über Phosphorpentoxid getrocknet.
Ausbeute: 3,10 g (80 % d.Th.).
DC: Kieselgel, Essigester; $R_f$=0,58.

b) Oestradiol-dimethylcarboxymethoxim-N-hydroxysuccinimidester 10

970 mg (2,5 mmol) der Carbonsäure 9 wurden mit 350 mg (3 mmol) N-Hydroxysuccinimid 4 in 30 ml trockenem THF gelöst und mit 620 mg (3 mmol) Dicyclohexylcarbodiimid in 10 ml THF versetzt. Man ließ 3 Stunden bei 20°C rühren, dann wurde vom ausgefallenen Dicyclohexylharnstoff abfiltriert und die Lösung im Wasserstrahlvakuum eingedampft. Der Rückstand wurde in 25 ml THF aufgelöst und die Lösung 16 Stunden bei 4°C abgekühlt. Dann wurde filtriert und das Lösungsmittel im Vakuum entfernt. Den öligen Rückstand digerierte man solange mit 20 ml Isopropanol, bis er sich vollkommen verfestigt hatte. Das Produkt 10 wurde abgesaugt, mit 50 ml Diisopropylether gewaschen und im Exsikkator über Phosphorpentoxid getrocknet.
Ausbeute: 940 mg (78 % d.Th.).
DC: Kieselgel RP-18, Nitromethan/Ethanol 9:1; $R_f$0,70.

c) Oestradiol-6-dimethylcarboxymethoxim-POD-Konjugat 11

Herstellung analog 7 (siehe Beispiel 1, 3).

## Patentansprüche

1. Haptenderivate der allgemeinen Formel (I)

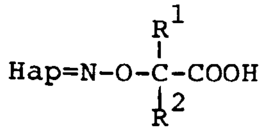

(I)

worin Hap der aus einem Keto- oder Aldehydgruppen tragenden Hapten durch Abspaltung einer Oxogruppe gebildete Rest ist, und $R^1$ und $R^2$ gleich oder verschieden sind und eine Niederalkylgruppe mit 1 bis 7, insbesondere 1 bis 3 Kohlenstoffatomen bedeuten, und ein Rest $R^1$ oder $R^2$ auch ein Wasserstoffatom sein kann.

2. Haptenderivate nach Anspruch 1, **dadurch gekennzeichnet,** daß das Hapten ein Steroidhormon ist.

3. Verfahren zur Herstellung von Haptenderivaten der allgemeinen Formel (I) nach Anspruch 1 oder 2, **dadurch gekennzeichnet,** daß man in einem eine Keto- oder Aldehydgruppe tragenden Hapten eine Keto- oder Aldehydgruppe mit einer Verbindung der Formel (II)

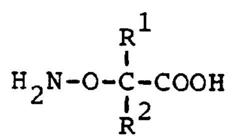

(II)

worin $R^1$ und $R^2$ die für Formel (I) angegebene Bedeutung besitzen, in an sich bekannter Weise in das entsprechende Oximderivat überführt.

4. Hapten-Protein-Konjugate der allgemeinen Formel (III)

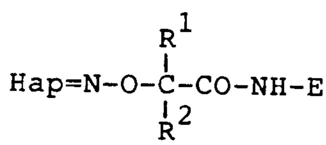

(III)

worin Hap, $R^1$ und $R^2$ die in Anspruch 1 angegebene Bedeutung besitzen, und E-NH- den Rest eines über eine ε-Aminogruppe eines Lysinrestes gebundenen Proteins bedeutet.

5. Hapten-Protein-Konjugat nach Anspruch 4, **dadurch gekennzeichnet,** daß das Hapten ein Steroidhormon ist.

6. Hapten-Protein-Konjugat nach Anspruch 4 oder 5, **dadurch gekennzeichnet,** daß das Protein ein Enzym ist.

7. Verfahren zur Herstellung eines Hapten-Protein-Konjugats der Formel (III) nach einem der Ansprüche 4 bis 6, **dadurch gekennzeichnet,** daß man ein Haptenderivat der Formel (I) gemäß einem der Ansprüche 1 oder 2 mit einem Protein $E\text{-}NH_2$, worin $-NH_2$ eine ε-Aminogruppe eines Lysinrestes bedeutet, auf an sich bekannte Weise umsetzt.

8. Verwendung eines Haptenderivats der Formel (I) nach einem der Ansprüche 1 oder 2 zur Herstellung von Hapten-Protein-Konjugaten der Formel (III) gemäß einem der Ansprüche 4 bis 6.

9. Verwendung eines Hapten-Protein-Konjugats nach einem der Ansprüche 4 bis 6 zur Immunisierung von zur Antikörperbildung geeigneten Organismen.

10. Verwendung eines Hapten-Protein-Konjugats nach Anspruch 6 als Markierungsenzym in einem Immunoassay.

## Claims

1. Hapten derivatives of the general formula (I)

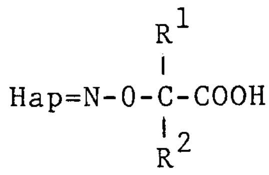

(I)

wherein Hap is a residue formed from a hapten carrying a keto or aldehyde group by splitting off of an oxo group and $R^1$ and $R^2$ are the same or different and signify a lower alkyl group with 1 to 7, especially 1 to 3 carbon atoms and one radical $R^1$ or $R^2$ can also be a hydrogen atom.

2. Hapten derivatives according to claim 1, characterised in that the hapten is a steroid hormone.

3. Process for the preparation of hapten derivatives of the general formula (I) according to claim 1 or 2, characterised in that, in a hapten carrying a keto or aldehyde group, one converts a keto or aldehyde group in per se known manner into the corresponding oxime derivative with a compound of the formula (II)

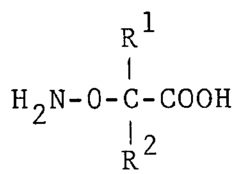

(II)

wherein $R^1$ and $R^2$ possess the meaning given for formula (I).

4. Hapten-protein conjugates of the general formula (III)

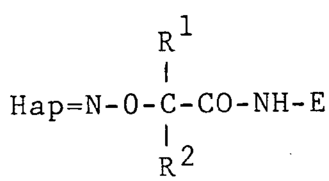

(III)

wherein Hap, $R^1$ and $R^2$ possess the meaning given in claim 1 and E-NH signifies the residue of a protein bound via an ε-amino group of a lysine residue.

5. Hapten-protein conjugate according to claim 4, characterised in that the hapten is a steroid hormone.

6. Hapten-protein conjugate according to claim 4 or 5, characterised in that the protein is an enzyme.

7. Process for the preparation of a hapten-protein conjugate of the formula (III) according to one of claims 4 to 6, characterised in that one reacts a hapten derivative of the formula (I) according to one of claims 1 or 2 in per se known manner with a protein $E-NH_2$, wherein $-NH_2$ signifies an ε-amino group of a lysine residue.

8. Use of a hapten derivative of the formula (I) according to one of claims 1 or 2 for the preparation of hapten-protein conjugates of the formula (III) according to one of claims 4 to 6.

9. Use of a hapten-protein conjugate according to one of claims 4 to 6 for the immunisation of organisms suitable for the formation of antibodies.

10. Use of a hapten-protein conjugate according to claim 6 as labelling enzyme in an immunoassay.

**Revendications**

1. Dérivés d'haptènes de formule générale (I)

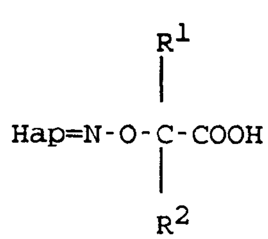

(I)

dans laquelle Hap représente le reste formé à partir d'un haptène porteur de groupes cétone ou aldéhyde par élimination d'un groupe oxo, et $R^1$ et $R^2$ sont identiques ou différents et représentent un groupe alkyle inférieur de 1 à 7, de préférence de 1 à 3 atomes de carbone, et un reste $R^1$ ou $R^2$ peut aussi représenter un atome d'hydrogène.

2. Dérives d'aptènes selon la revendication 1, caractérisés en ce que l'haptène est une hormone stéroïde.

3. Procédé de préparation de dérivés d'haptènes de formule générale (I) selon la revendication 1 ou 2, caractérisé en ce que, dans un haptène porteur d'un groupe cétone ou aldéhyde, on fait réagir un groupe cétone ou aldéhyde avec un composé de formule (II)

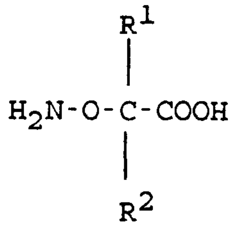

(II)

dans laquelle $R^1$ et $R^2$ ont la signification donnée pour la formule (I) pour former de manière connue en soi le dérivé d'oxime correspondant.

4. Conjugués haptène-protéine de formule générale (III)

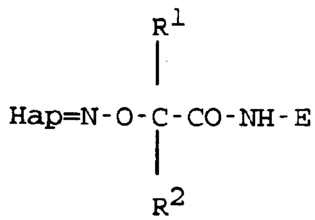

(III)

dans laquelle Hap, $R^1$ et $R^2$ ont la signification donnée dans la revendication 1 et E-NH- représente le reste d'une protéine liée par un groupe amine en ε d'un reste lysine.

5. Conjugué haptène-protéine selon la revendication 4, caractérisé en ce que l'haptène est une hormone stéroïde.

6. Conjugue haptène-protéine selon la revendication 4 ou 5, caractérisé en ce que la protéine est une enzyme.

7. Procédé de préparation d'un conjugué haptène-protéine de formule (III) selon l'une des revendications 4 à 6, caractérisé en ce que l'on fait réagir, de manière connue en soi, un dérivé d'haptène de formule (I) selon l'une des revendications 1 ou 2 avec une protéine $E\text{-}NH_2$ dans laquelle $-NH_2$ représente un groupe amine en ε d'un reste lysine.

8. Utilisation d'un dérivé d'haptène de formule (I) selon l'une des revendications 1 ou 2 pour la préparation de conjugués haptène-protéine de formule (III) selon l'une des revendications 4 à 6.

9. Utilisation d'un conjugué haptène-protéine selon l'une des revendications 4 à 6 pour l'immunisation d'organismes convenant à la formation d'anticorps.

10. utilisation d'un conjugue haptène-protéine selon la revendication 6 comme enzyme de marquage dans un dosage immunologique.

FIG.1

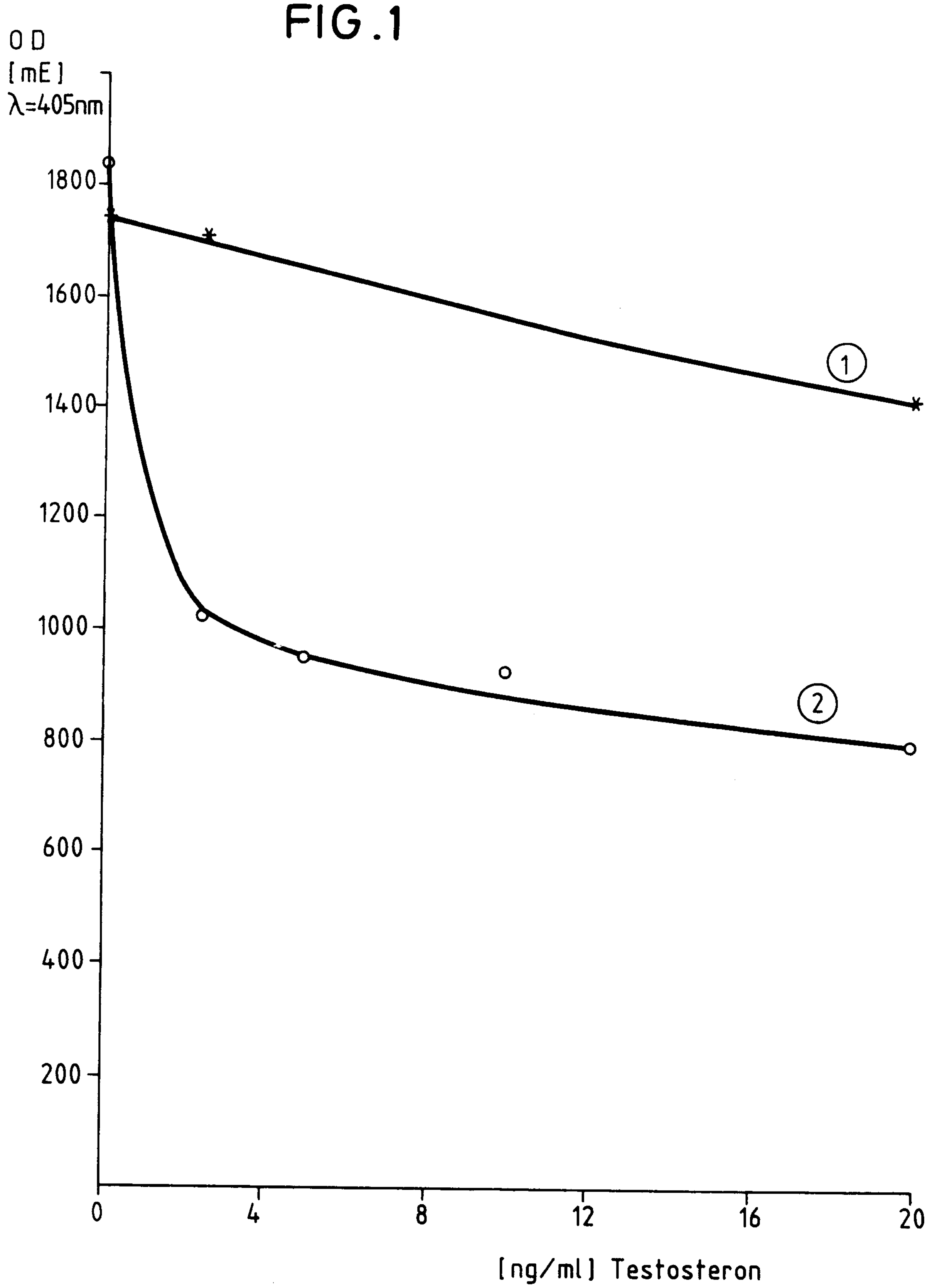
OD
[mE]
λ=405nm
1800
1600
1400
1200
1000
800
600
400
200
0
4
8
12
16
20
1
2
[ng/ml] Testosteron